Europäisches Patentamt

(19) European Patent Office

Office européen des brevets

(11) Publication number: **0 246 086**
**A2**

## (12) EUROPEAN PATENT APPLICATION

(21) Application number: **87304253.5**

(22) Date of filing: **13.05.87**

(51) Int. Cl.4: **A 61 B 17/02**

(30) Priority: **14.05.86 AR 303956**

(43) Date of publication of application:
**19.11.87 Bulletin 87/47**

(84) Designated Contracting States:
**AT BE DE ES FR GB IT**

(71) Applicant: **Kleiman, Aldo Sergio**
**9 de Julio 288**
**Concepcion del Uruguay Prov. of Entre Rios 3260 (AR)**

(72) Inventor: **Kleiman, Aldo Sergio**
**9 de Julio 288**
**Concepcion del Uruguay Prov. of Entre Rios 3260 (AR)**

(74) Representative: **Pendlebury, Anthony et al**
**Page, White & Farrer 5 Plough Place New Fetter Lane**
**London EC4A 1HY (GB)**

(54) **A procedure for carrying out a surgical operation and a retracting laparoscope for separating organs in surgery.**

(57) A retracting laparoscope (1, 20) for use in surgery and more particularly adapted for carrying out a surgical operation comprising a laparoscopic cholecystectomy. The laparoscope comprises a base member (4, 21) including a window-like opening (5) defining an edge-portion therearound, at least one pair of spaced-apart arms (2) projecting through the window-like opening, which arms are capable of being inserted through a 15 mm incision made in the human body, the arms being swingably supported by the edge-portion of the window-like opening and swingable along predetermined paths in opposite directions to place and maintain portions of human organs spaced-apart, for obtaining a good exposure of the operating zone, and actuating means (11) mounted in said base member for locating and blocking said arms in any position of their respective paths. A procedure for carrying out a surgical operation, by means of the use of the retracting laparoscope, comprising the steps of performing a 15 mm incision in the human body, introducing the laparaoscope therethrough, actuating the laparoscope in order to open its arms to have a good exposure of the operation zone, and performing the surgical operation.

FIG.3

FIG.4

EP 0 246 086 A2

## Description

**"A PROCEDURE FOR CARRYING OUT A SURGICAL OPERATION AND A RETRACTING LAPAROSCOPE FOR SEPARATING ORGANS IN SURGERY"**

### Technical Field of the Invention:

The present invention relates to a procedure for carrying out a surgical operation as well as to a retracting laparoscope for use in surgery and more particularly adapted for carrying out a surgical operation comprising laparoscopic cholecystectomy. This laparoscope instrument is especially indicated for certain surgical operations, complementing conventional surgery instruments, the aim of which is to provide a good exposure of the operating zone within the human body.

The present instrument is particularly applicable for separating and maintaining in spaced-apart position certain human organs, specially the liver and the intestinal mass, to facilitate surgery such as "cholecystectomy by minilaparotomy or laporoscopic cholecystectomy".

### Background Art:

Although worldwide the surgical trend appears to develop new procedures for decreasing morbidity, mortality and hospitalization cost, in some particular surgical operations it has been always necessary to perform large incisions in order to provide the necessary exposure of the organs to be treated, for example in biliary surgery.

In the particular field of biliary surgery, multiple retrograde endoscopic procedures are currently in use as well as through percutaneous approach under radioscopic control. As to laparoscopic surgery there has been considerable progress in the area of gynecology with adequate manoeuvers through second and third sheet punctions. Laparoscopy or oöphorectomy, miomectomy and salpingotomy have been reported.

Of course, surgery in the area of gynecology is facilitated because by lying the human body in ther pneumoperitoneum and Trendelenburg position, a good exposure is provided for surgery. In this position, the intestinal mass is moved in the direction towards the head of the human body which therefore leaves the gynecologic zone to be treated free of obstaculizing organs. Then an incision is made for the introduction of a laparoscope for viewing the operation zone to allow the doctor to effect the surgery in said zone by means of at least two punctions adjacent the incision and through which the needed instruments are introduced for operation.

Although the above technique facilitates gynecological surgery, it is not useful in biliary surgery because of the uncapability of organs to move apart from the liver in order to provide a good exposure of the gall-bladder for resecting the same.

In the field of biliary surgery, it is common to perform a long incision, for example of about 15 cm. Such an incision is necessary to permit the introduction within the human body of considerably large separating shells which separate the intestinal mass and the liver and leave the gall-bladder exposed for surgery. Obviously, the need of such a long incision can bring about a traumatic effect on the patient.

### Disclosure of the invention:

Bearing in mind the laparoscopic surgery utilized in gynecology, by means of which it is possible to remove miomas, tubes and ovarios by laporoscopy, I have studied the possibility of resecting the gall-bladder by means of a similar technique. However as stated above, many obstacles were found in respect of the organs which are present in the zone to be operated upon due to the lack of suitable instruments to carry out such a technique.

Therefore the present invention provides a device for use in biliary surgery which enables such a surgical technique to be followed for performing laporoscopic cholecystectomy or, in other words cholecystectomy through minilaparotomy. The device of the present invention comprises a retracting laparoscope capable of retracting the viscus in such a fashion so as to provide ample and good surgical exposure and also capable of being combined with other instruments for surgical manoeuvers through second and third sheet punction.

The invention also provides a procedure for carrying out surgical operations minimizing traumatic effects in the patient. The procedure is particularly designed for uncomplicated gall-bladder lithiasis and cholecystosis, and will favour patients particularly at risk with conventional laparotomy, e.g.: enphisema, asthma, chronic bronchitis, chronic respiratory failure in general, and cardiac failure.

Furthermore, the device of the present invention provides the necessary means for illuminating the operating zone. These means may consist of cold light, optical fibres, etc.

The laparoscope of the present invention has the advantage that only a minor incision of about 15 mm is necessary to allow its introduction, in a closed position, into the human body. Although the portion of the laparoscope which is to be located over this incision has a size compatible with said incision, i.e. approximately 20 mm, the members of the laparoscope used to separate the relevant human organs are capable of adopting and maintaining, at an open position within the human body, a separation of at least 15 cm measured between the free ends of the separating members or arms of the laparoscope.

In one aspect the invention provides a procedure for carrying out a surgical operation, specially adapted for resecting the gall-bladder, comprising the steps of applying general anaesthesia to the patient, positioning the patient in a substantially horizontal position; performing a minilaparotomy by effecting a subcostal right side, transverse midclavicular incision of approximately 15 mm in length; performing the elevation of the gall-bladder fundus and the division of peritoneal adhesions; setting the retracting laparascope along the ventral aspect of the gall-bladder and cephalad to the intestinal mass; drawing apart the retracting laparoscope arms thus exposing the full length of the gall-bladder; introducing two sheaths, one on each side of the minilaparotomy through stub wounds; emptying the gall-bladder with syringe; separating the gall-bladder from its liver bed with diathermy and scissors; ligating the cystic duct with ligature or clip applicator and division of same; removing the gall-bladder through minilaparotomy and closuring the same.

According to another aspect, the invention provides a retracting laparoscope for separating organs in surgery, insertable in a previously made incision in the human body, capable of maintaining portions of human organs in spaced-apart positions, to thus gain visual access to deeper located areas where an operation or a direct treatment is to be performed, said retracting laparoscope comprising a base member including a window-like opening defining an edge-portion, at least one pair of spaced-apart arms projecting through said window-like opening, said arms being swingably supported by said edge-portion and swingable along predetermined paths in opposite directions, and actuating means mounted on one face of said base member to position and block said arms in any position of their corresponding paths.

According to yet another aspect, the invention provides a retracting laparoscope for separating organs in surgery, insertable in a previously made incision in the human body, capable of maintaining portions of human organs in spaced-apart positions, to thus gain visual access to deeper located areas where an operation or a direct treatment is to be performed, said retracting laparascope comprising a base member including a window-like opening defining an edge-portion, at least four spaced-apart arms projecting through said window-like opening, said arms being swingably supported by said edge-portion and swingable along predetermined paths in opposite directions, each arm being associated with a respective lever portion defining a free end including a threaded opening, a threaded rod mounted on one face of said base member extending through the threaded opening of an associated lever portion, each rod having a slidable base slidably retained in respective guide means mounted on the base member, each rod including an actuating head at the end opposite to the respective slidable base.

In still another aspect, the invention provides a retracting laparoscope for separating organs in surgery, insertable in a previously made incision in the human body, capable of maintaining portions of human organs in spaced-apart position, to thus gain visual access to deeper located areas where an operation or a direct treatment is to be performed, said retracting laparoscope comprising a base member consisting of two articulated parts and including at the zone of the articulation a window-like opening defining a first edge and two second edges which form an acute angle therebetween, the three edges defining a triangle-like configuration for the window-like opening, at least four spaced-apart arms projecting through said window-like opening, a first two of said arms being parallel to each other and swingably supported by said first edge, the other, second, two arms being each swingably supported by each of said second two edges, the arms being swingable along predetermined paths in opposite directions, the first two arms being always parallel to each other, the second two arms being capable of separating from each other like a fan and maintaining the same distance from each other at a zone wherein they are articulated with the corresponding second edge-portions of the window-like opening, and actuating means mounted on one face of said base member for positioning and blocking said arms in any position of their respective paths, said arms being associated with respective lever portions located at a side of the base member corresponding to said face, the lever portions being connected to said actuating means.

For a better understanding of the present invention, reference will now be made, by way of example, to the enclosed drawings, in which two laparoscope embodiments are disclosed, which facilitate the comprehension of the principle of the invention.

Brief description of the drawings:

Figure 1 shows an upper perspective view of one embodiment of the laparoscope of the invention.

Figure 2 shows a lower perspective view of the retracting laparoscope of figure 1.

Figure 3 is an upper perspective view of a second embodiment of the retracting laparoscope of the present invention in a closed position.

Figure 4 shows a partial longitudinal section along line IV-IV of figure 3.

Figure 5 shows an upper perspective view of the laparoscope of figures 3 and 4, but in an open or retracting position.

Figure 6 shows a partial longitudinal section of the laparoscope of figure 5 taken along line VI-VI.

Figure 7 is a lower perspective view of the laparoscope of figures 3 to 6.

Figure 8 is a perspective view of the laparoscope of figures 3 to 7, but partially disassembled for cleaning.

Detailed Account of Examples of the Invention:

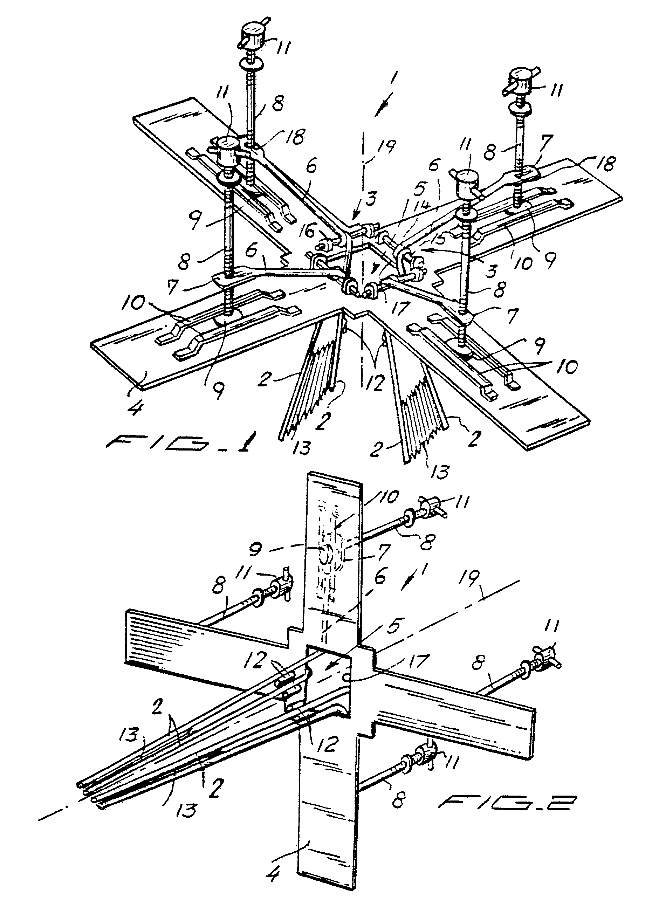

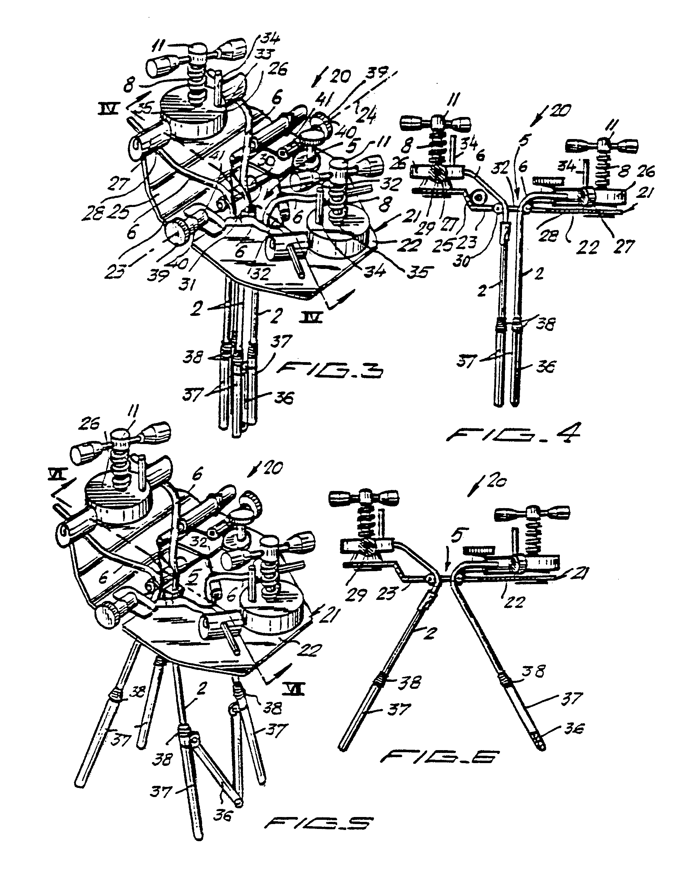

Figures 1 and 2 show one embodiment of the laparoscope of the present invention.

The laparoscope of figures 1 and 2 is generally indicated by reference numeral 1. It basically comprises a base member 4 providing a window-like opening, particularly at the centre thereof. In this embodiment, the base member has a cross configuration, but it is obvious that it may comprise other forms as well as structures provided that it will be able to support the pertinent members described hereinbelow.

At least two, but preferably four, separating or retracting arms 2 are pivotally mounted on the base member 4. The arms are joined to an acting lever portion 6 and between the associated arm and lever portion there is provided a pivot connection 3. In this particular embodiment, the pivot connection 3 comprises a loop portion 14 of the wire constituting arms 2 and lever portions 6, said loop portion 14 being arranged around a shaft 15 mounted on base member 4 and retained by end supports 16. Shaft 15 is located parallel and adjacent edge-portion 17 which defines window-like opening 5.

At the free ends of associated arms 2 there may be provided a joining member, which in this particular case comprises a foldable membrane 13 which, upon retraction of the arms to adopt the open position (shown in figure 1) is unfolded in order to have a larger size. Of course, this membrane 13 may be replaced by another member which is capable of increasing its length as associated arms 2 increase their separation at the free ends thereof.

At least one of arms 2 may include any suitable kind of light emitter such as cold-light emitter 12 or optical fibres. In any case, although not illustrated in the drawings, the electrical source will be an external source connected by means of respective wires to the corresponding light emitter 12. The wires are not illustrated in the figures in order to facilitate an interpretation of the drawings.

As stated above, arms 2 project through lever portions 6 which in turn end in respective end-portions 7. Each end-portion has a threaded through opening 18 which is coupled to a corresponding threaded rod 8 along which end portion 7 will move in response to the rotation of rod 8 achieved by means of actuating head 11. Rod 8 is slidably mounted on base 4 by means of the slidable connection between a movable base 9 which is fixed to rod 8 and which is slidably retained between guides 10 fixed to base member 4.

Due to the slidable retention of base member 9 between guides 10 when the rod 8 is rotated in the proper direction, portion 7 will move up along rod 8 and therefore lever portion 6 and arm 2 will pivot on pivot connection 3. In order to permit the end-portion 7 to move up along the rod 8, it is necessary for the rod 8 to move against the pivot connection 3, and this will occur when the movable base 9 moves between the guides 10 towards the pivot connection 3. During this movement, the arms 2 will separate from each other along a virtual central point coincident with the longitudinal axis 19 of the window-like opening 5.

During movement in the opposite direction, in order to place arms 2 in a closed position whereby arms 2 converge towards virtual axis 19, end-portion 7 will move down along rod 8 and as a result of this, movable base 9 will move away from pivot connection 3 between guides 10.

Upon carrying out an operation using the laparoscope of the invention, for example in biliary surgery to resect the gall-bladder, it is necessary to perform an incision of about 15 mm. Laparoscope 1 will have its arms 2 in closed position as shown in figure 2, i.e. with the arms adjacent to virtual longitudinal axis 19. In this position the arms are introduced into the human body through the incision, and then acting heads 11 are actuated so as to cause the arms 2 to open, in order to separate the intestinal mass and the liver to obtain a good exposure of the gall-bladder to be resected. Although arms 2 will separate from each other, the distance therebetween at the first connection 3 will be maintained.

Through pertinent punctions, surgery of the gall-bladder may be effected by means of appropriate instruments, while the surgeon has a good view of the operating area which up to now was impossible in biliary surgery by means of conventional laparoscopes.

It is obvious that the present laparoscope may be combined with a video-camera in order to carry out the surgical operation viewing the operating zone through a television set.

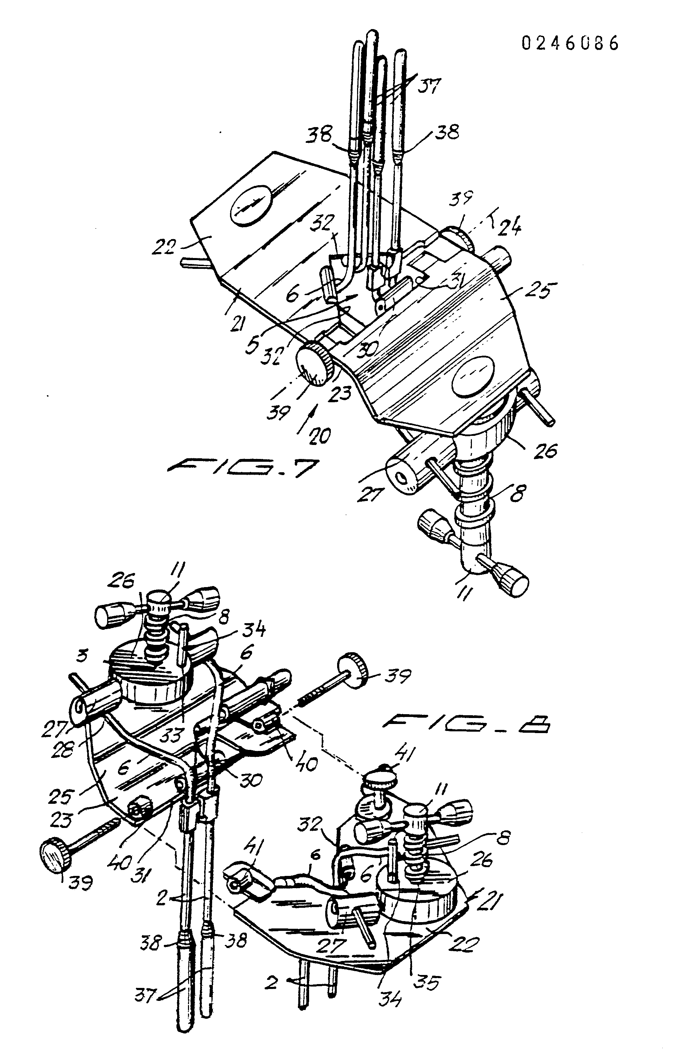

In figures 3 to 8 a second embodiment of the present retracting laparoscope is shown. This second embodiment is basically the same as the first embodiment shown in figures 1 and 2, except that some structural and operating members have been partially modified.

For the same basic members the same reference numerals have been retained and new reference numerals are used for new elements.

Laparoscope 20, representing the second embodiment of the invention, comprises base member 21 which basically has the same function as base member 4 of the embodiment shown in figures 1 and 2, but differs therefrom in configuration and structure. Base member 21 comprises two parts 22 and 23 which are articulated along longitudinal axis 24.

Between the two parts 22 and 23, there is a window-like opening 5 which in this case has a triangle-like configuration, in order to obtain a different effect in the opening of arms 2. Part 23 of plate 21 includes a step portion 25 which permits plate 21 to be adapted to the human body profile, specially at the costal part of the human body where the rib system ends.

Although threaded rod 8 as well as acting head 11 are similar as in the embodiment of figures 1 and 2, the mechanism by means of which the lever portions and arms 2 are actuated is modified. Each free end of lever portions 6 is slidably retained within opening 28 formed in pivot support 27 to permit accommodation of lever portions 6 during movement of the mechanism. Support 27 is rotatable connected to annular hub 26 by means of shaft 29 (see figure 4).

Annular hub 26 has a through opening 35 which is internally threaded and is threadably coupled to threaded rod 8. Furthermore, hub 26 has an opening 33 through which column 34 extends. Column 34 is fixedly joined to base member 21. It is readily understood that when acting head 11 is operated to rotate rod 8, and when lever portions 6 and arms 2 are in the position shown in figures 3 and 4, annular hub 26 will move up along rod 8 due to the fact that column 34 avoids rotation of hub 26, so that arms 2 will open relative to each other to adopt the position shown in figures 5 and 6.

In view of the triangle-like configuration of window 5, the behaviour of each pair of arms 2, when moving to a spaced-apart position, will be different. Arms 2, located on the left-hand side of figures 4 and 6, will move keeping parallel to each other, because they are articulated on the same edge-portion 31, whilst the arms shown at the right-hand side of the above cited figures will move in such a manner that they will separate outwardly as shown in figure 5. This is so because each lever portion 6 and arm 2 on the right-hand side is articulated in different edge-portions 32 which form an angle. In both cases the pivot connection between arms 2 and base member 21 is constituted by bushing 30 which is mounted around a shaft (not shown) connected to base member 21. Arms 2, capable of moving in a parallel configuration, are mounted on the same bushing 30, and arms 2, capable of moving outwardly relative to each other (to be located spaced-apart in an open configuration), are each one connected to a different shaft 32 forming an angle.

In order to obtain the particular different movement of arms 2, lever supports 27 of parallel arms 2 will be diametrically arranged on the annular hub 26 (at the left-hand side in figures 4 and 6), whilst lever supports 27 of the mechanism actuating the arms capable of separating from each other are mounted on annular tube 26 forming an angle therebetween (see the right-hand side of figures 4 and 6). This angle between supports 27 will be determined on the basis of the separation desired between arms 2.

In some cases it may be necessary for the arms capable of maintaining a parallel configuration not to open in a perpendicular direction relative to edge-portion 31. Instead, it may be necessary for arms 2 to open in a direction forming an angle with the direction parallel to edge-portion 31. In this case, shaft 30 should be a split shaft, one for each arm 2, and both shafts 30 should form an angle with edge-portion 31.

Arms 2 which open in a spaced-apart configuration, may include, connected at the zone of the free ends thereof, a metallic fan-like member 36 which is capable of unfolding when arms 2 move and separate. The function of this member 36 is to more effectively retain the intestinal mass during a surgery for resecting a gall-bladder.

In a preferred embodiment, this member 36 will be mounted and connected to complementary telescopic arms 37 which are telescopically mounted on each of arms 2 in order to permit the length of the arms to increase depending on the configuration of the patient's body. It should be understood that the abdomen of each patient varies in volume, and therefore the depth wherein the organs are located is different for each patient. Telescopic-arm 37 is capable of being slidably moved along corresponding arm 2 and locked in a predetermined position by means of retaining nuts 38.

As stated above, base member 21 is constituted by two parts 23 and 24. Both parts are articulated along longitudinal axis 24 by means of screws 39 passing through bushing portions 41, each one corresponding to parts 24 and 23. In order to maintain parts 23 and 24 in the same plane, tags 42 are provided, each one joined to each base part and one of which is blockable by means of retaining cam 43, operable through acting screw 44 mounted on base part 23.

In view of the above-described structure, base parts 23 and 24 may be split from the common plane thereof and along axis 24. This facilitates the removal of arms 2 from the human body, thus avoiding that the legs hook onto the intestinal mass or any organs in contact therewith.

In addition, this pivot connection between parts 23 and 24 permits the disassembly of base member 21 into two parts as shown in figure 8. This is useful for cleaning the laparoscope after a surgical operation.

As stated above, the present invention also provides a procedure for carrying out a surgical operation such as for example to resect the gall-bladder. This procedure or technique for resecting the gall-bladder may be carried out using any laparoscope described hereinabove, and obviously may be carried out with any laparoscope of the same kind intended to broaden the operation zone by separating the organs which represent an obstacle for the surgical operation.

Thus, the present invention provides a procedure for carrying out a surgical operation, specially adapted for resecting the gall-bladder, comprising the steps of applying general anaesthesia to the patient, positioning the patient in a substantially horizontal position; performing a minilaparotomy by effecting a subcostal right side transverse midclavicular incision of approximately 15 mm in length; performing the elevation of the gall-bladder fundus and the division of peritoneal adhesions; setting the retracting laparascope along the ventral aspect of the gall blader and cephalad to the intestinal mass; drawing apart the retracting laparoscope arms thus exposing the full length of the gall-bladder; introducing two sheaths, one on each side of the minilaparotomy through stub wounds; emptying the gall-bladder with a syringe; separating the gall-bladder from its liver bed with diathermy and scissors; ligating the cystic duct with ligature or clip applicators and dividing same; removing the gall-bladder through the minilaparotomy and closing the same.

Although the essential features of the invention have been described by means of a preferred embodiment, the invention is not limited to this embodiment and extends instead to all alternative forms within the scope of the appended claims.

**Claims**

1. Procedure for carrying out a surgical operation, specially adapted for resecting the gall-bladder, characterized by comprising the steps of applying general anaesthesia to the patient, positioning the patient in a substantially horizontal position; performing a minilaparotomy by effecting a subcostal right side transverse mid-clavicular incision of approximately 15 mm in length; performing the elevation of the gall-bladder fundus and division of peritoneal adhesions; setting a retracting laparascope (1) along the ventral aspect of the gall blader and cephalad to the intestinal mass; drawing apart the retracting laparoscope arms (2) thus exposing the full length of the gall-bladder; introducing two sheaths, one on each side of the minilaparotomy through stub wounds; emptying the gall-bladder with syringe; separating the gall-bladder from its liver bed with diathermy and scissors; ligating the cystic duct with ligature or clip applicator and division of same; removing the gall-bladder through the minilaparotomy and closing the same.

2. A retracting laparoscope (1) for separating organs in surgery, insertable in a previously made incision in the human body, capable of maintaining portions of human organs in spaced-apart position to thus gain visual access to deeper located areas where an operation or a direct treatment is to be performed, said retracting laparoscope characterized by comprising a base member (4) including a window-like opening (5) defining an edge-portion (17), at least one pair of spaced-apart arms (2) projecting through said window-like opening, said arms being swingably supported by said edge-portion and swingable along predetermined paths in opposite directions, and actuating means mounted on one face of said base member for positioning and blocking said arms in any position of their respective paths.

3. A retracting laparoscope as in claim 2, characterized in that at least two of said arms are extendable by means of respective telescopic-arms (37), each telescopic-arm is slidable along the corresponding arm and is adapted to be locked in a predetermined position by retaining nuts (38).

4. A retracting laparoscope (1) for separating organs in surgery, insertable in a previously made incision in the human body, capable of maintaining portions of human organs in spaced-apart position to thus gain visual access to deeper located areas where an operation or a direct treatment is to be performed, said retracting laparoscope characterized by comprising a base member (4) including a window-like opening (5) defining an edge-portion (17), at least four spaced-apart arms (2) projecting through said window-like opening, said arms being swingably supported by said edge-portion and swingable along predetermined paths in opposite directions, each arm being assosciated with a respective lever portion defining a free end including a threaded opening (18), a threaded rod (8) mounted on one face of the base member extending through the threaded opening of an assosciated lever portion (6), each rod having a slidable base (9) slidably retained in respective guide means (10) mounted in the base member, each rod including an actuating head (11) at the end opposite to the respective slidable base.

5. A retracting laparoscope as in claim 4, characterized in that said arms are assosciated in pairs and each pair is connected through an extendable membrane (13).

6. A retracting laparoscope as in claim 4, characterized in that said arms are assosciated in pairs and each pair is connected through an extendable fan-like member (36).

7. A retracting laparoscope (20) for separating organs in surgery, insertable in a previously made incision in the human body, capable of maintaining portions of human organs in spaced-apart position to thus gain visual access to deeper located areas, where an operation or a direct treatment is to be performed, said retracting laparoscope characterized by comprising a base member (21) consisting of two articulated parts (21, 22) and including at the zone of the articulation a window-like (5) opening defining a first edge (31) and two second edges (32) which form an acute angle therebetween, the three edges defining a triangle-like configuration for the window-like opening, at least four spaced-apart arms (2) projecting through said window-like opening, a first two of said arms being parallel to each other and swingably supported by said first edge, the other second two arms being each swingably supported by each one of said second two edges, the arms being swingable along predetermined paths in opposite directions, the first two arms being always parallel to each other, the second two arms being capable of separating from each other like a fan and maintaining the same distance from each other at a zone wherein they are articulated to the corresponding second edge-portions of the window-like opening, and actuating means (11) mounted on one face of said base member for positioning and blocking said arms in any position of their respective paths, said arms being assosciated with respective lever portions (6) located at a side of the base member corresponding to said face, the lever portions being connected to said actuating means.

8. A retracting laparoscope as in claim 7, characterized in that said arms are assosciated in pairs and one pair, formed by said second two arms, is connected through an extendable membrane (13).

9. A retracting laparoscope as in claim 7, characterized in that said arms are assosciated in pairs and one pair, formed by said second two arms, is connected through an extendable fan-like member (36).

10. A retracting laparosocope as in any of claims 2 to 10, characterized in that at least one arm includes a light emitter (12).

11. A retracting laparoscope as in claim 10, characterized in that said emitter is a cold light emitter (12).

12. A retracting laparoscope as in claim 10, characterized in that said emitter is an optical fibre emitter.

13. A retracting laparoscope as in claim 11, characterized in that said base member comprises two parts pivotally connected to each other along a longitudinal axis (24) parallel to said first edge, the two parts having locking means for retaining both parts in a common plane.

14. A retracting laparoscope as in claim 13, characterized in that said both parts are connected by a detachable pivot connection allowing the parts to be separated for cleaning.

15. A retracting laparoscope as in claim 14, characterized in that one of said parts of the base member includes a step portion (25) which permits the base member to be adapted to the human body profile.

16. A retracting laparoscope as in claim 4 or 7, characterized in that at least two of said arms are extendable by means of respective telescopic-arms (37), each telescopic-arm being slidable along the corresponding arm and is adapted to be locked in a predetermined position by retaining nuts (38).

17. Procedure for carrying out a surgical operation, by means of the use of the retracting laparascope of any of claims 2 to 16.

0246086

FIG_1

FIG.2

0246086

FIG.3

FIG.4

FIG.5

FIG.6

0246086

FIG.7

FIG.8